# EUROPÄISCHE PATENTSCHRIFT

(11) **EP 1 937 839 B1**
(45) Veröffentlichungstag und Bekanntmachung des Hinweises auf die Patenterteilung: **21.09.2011**
(21) Anmeldenummer: 06792341.7
(22) Anmeldetag: 30.09.2006
(51) Int. Cl.: C12Q 1/68

(54) **VERFAHREN ZUR DIAGNOSE THROMBOEMBOLISCHER ERKRANKUNGEN UND KORONARER HERZERKRANKUNGEN**
METHOD FOR DIAGNOSING THROMBOEMBOLIC DISORDERS AND CORONARY HEART DISEASES
PROCEDE POUR DIAGNOSTIQUER DES MALADIES THROMBOEMBOLIQUES ET DES MALADIES CARDIAQUES CORONARIENNES

(30) Priorität: 12.10.2005 DE 102005048899
(43) Veröffentlichungstag der Anmeldung: 02.07.2008
(73) Patentinhaber: SANOFI, 75013 Paris (FR)
(72) Erfinder: KOZIAN, Detlef, 65926 Frankfurt am Main (DE); HERRMANN, Matthias, 65926 Frankfurt am Main (DE)
(74) Vertreter: Schneider, Rudolf
(86) Internationale Anmeldenummer: PCT/EP2006/009517
(87) Internationale Veröffentlichungsnummer: WO 2007/042165

(56) Entgegenhaltungen:
- WO-A2-2004/058052
- TAYLOR M S: "Characterization and comparative analysis of the EGLN gene family" GENE: AN INTERNATIONAL JOURNAL ON GENES AND GENOMES, ELSEVIER, AMSTERDAM, NL, Bd. 275, Nr. 1, 5. September 2001 (2001-09-05), Seiten 125-132, XP004307119 ISSN: 0378-1119

### SEQUENCE LISTING

<110> sanofi-Aventis Deutschland GmbH
<120> Verfahren zur Diagnose thromboembolische Erkrankungen und koronarer Herzerkrankungen
<130> DE 2005/049
<140>
   <141>
<160> 4
<170> PatentIn ver. 2.1
<210> 1
   <211> 2111
   <212> DNA
   <213> Homo sapiens
<400> 1
   gctttcccct gcctgcctgt ctctagtttc tctcacatcc cttttttttt ttcctttctc 60
   tagccaccct gaagggtccc ttcccaagcc cttagggacc gcagaggact tggggaccag 120
   caagcaaccc ccagggcacg agaagagctc ttgctgtctg ccctgcctca ccctgcccca 180
   cgccaggccc ggtggccccc agctgcatca agtggaggcg gaggaggagg cggaggaggg 240
   tggcaccatg ggcccgggcg gtgccctcca tgcccggggg atgaagacac tgctgccatg 300
   gacagcccgt gccagccgca gcccctaagt caggctctcc ctcagttacc agggtcttcg 360
   tcagagccct tggagcctga gcctggccgg gccaggatgg gagtggagag ttacctgccc 420
   tgtcccctgc tcccctccta ccactgtcca ggagtgccta gtgaggcctc ggcagggagt 480
   gggaccccca gagccacagc cacctctacc actgccagcc ctcttcggga cggttttggc 540
   gggcaggatg gtggtgagct gcggccgctg cagagtgaag gcgctgcagc gctggtcacc 600
   aaggggtgcc agcgattggc agcccagggc gcacggcctg aggcccccaa acggaaatgg 660
   gccgaggatg gtggggatgc cccttcaccc agcaaacggc cctgggccag gcaagagaac 720
   caggaggcag agcgggaggg tggcatgagc tgcagctgca gcagtggcag tggtgaggcc 780
   agtgctgggc tgatggagga ggcgctgccc tctgcgcccg agcgcctggc cctggactat 840
   atcgtgccct gcatgcggta ctacggcatc tgcgtcaagg acagcttcct gggggcagca 900
   ctgggcggtc gcgtgctggc cgaggtggag gccctcaaac ggggtgggcg cctgcgagac 960
   gggcagctag tgagccagag ggcgatcccg ccgcgcagca tccgtgggga ccagattgcc 1020
   tgggtggaag gccatgaacc aggctgtcga agcattggtg ccctcatggc ccatgtggac 1080
   gccgtcatcc gccactgcgc agggcggctg ggcagctatg tcatcaacgg gcgcaccaag 1140
   gccatggtgg cgtgttaccc aggcaacggg ctcgggtacg taaggcacgt tgacaatccc 1200
   cacggcgatg ggcgctgcat cacctgtatc tattacctga atcagaactg ggacgttaag 1260
   gtgcatggcg gcctgctgca gatcttccct gagggccggc ccgtggtagc caacatcgag 1320
   ccactctttg accggttgct cattttctgg tctgaccggc ggaaccccca cgaggtgaag 1380
   ccagcctatg ccaccaggta cgccatcact gtctggtatt ttgatgccaa ggagcgggca 1440
   gcagccaaag acaagtatca gctagcatca ggacagaaag gtgtccaagt acctgtatca 1500
   cagccgccta cgcccaccta gtggccagtc ccagagccgc atggcagaca gcttaaatga 1560
   cttcaggaga gccctgggcc tgtgctggct gctccttccc tgccaccgct gctgcttctg 1620
   actttgcctc tgtcctgcct ggtgtggagg gctctgtctg ttgctgagga ccaaggagga 1680
   gaagagacct ttgctgcccc atcatggggg ctggggttgt cacctggaca gggggcagcc 1740
   gtggaggcca ccgttaccaa ctgaagctgg gggcctgggt cctaccctgt ctggtcatga 1800
   ccccattagg tatggagagc tgggaggagg cattgtcact tcccaccagg atgcaggact 1860
   tggggttgag gtgagtcatg gcctcttgct ggcaatgggg tgggaggagt acccccaagt 1920
   cctctcactc ctccagcctg gaatgtgaag tgactcccca acccctttgg ccatggcagg 1980
   caccttttgg actgggctgc cactgcttgg gcagagtaaa aggtgccagg aggagcatgg 2040
   gtgtggaagt cctgtcagcc aagaaataaa agtttacctc agagctgcaa aaaaaaaaaa 2100
   aaaaaaaaaa a                                                     2111
<210> 2
   <211> 407
   <212> PRT
   <213> Homo sapiens
<400> 2
   Met Asp Ser Pro Cys Gln Pro Gln Pro Leu Ser Gln Ala Leu Pro Gln
   1 5 10 15
   Leu Pro Gly Ser Ser Ser Glu Pro Leu Glu Pro Glu Pro Gly Arg Ala
               20                  25                  30
   Arg Met Gly Val Glu Ser Tyr Leu Pro Cys Pro Leu Leu Pro Ser Tyr
           35                  40                  45
   His Cys Pro Gly Val Pro Ser Glu Ala Ser Ala Gly Ser Gly Thr Pro
       50                  55                  60
   Arg Ala Thr Ala Thr Ser Thr Thr Ala Ser Pro Leu Arg Asp Gly Phe
   65                  70                  75                  80
   Gly Gly Gln Asp Gly Gly Glu Leu Arg Pro Leu Gln Ser Glu Gly Ala
                   85                  90                  95
   Ala Ala Leu Val Thr Lys Gly Cys Gln Arg Leu Ala Ala Gln Gly Ala
               100                 105                 110
   Arg Pro Glu Ala Pro Lys Arg Lys Trp Ala Glu Asp Gly Gly Asp Ala
           115                 120                 125
   Pro Ser Pro Ser Lys Arg Pro Trp Ala Arg Gln Glu Asn Gln Glu Ala
       130                 135                 140
   Glu Arg Glu Gly Gly Met Ser Cys Ser Cys Ser Ser Gly Ser Gly Glu
   145                 150                 155                 160
   Ala Ser Ala Gly Leu Met Glu Glu Ala Leu Pro Ser Ala Pro Glu Arg
                   165                 170                 175
   Leu Ala Leu Asp Tyr Ile Val Pro Cys Met Arg Tyr Tyr Gly Ile Cys
               180                 185                 190
   Val Lys Asp Ser Phe Leu Gly Ala Ala Leu Gly Gly Arg Val Leu Ala
           195                 200                 205
   Glu Val Glu Ala Leu Lys Arg Gly Gly Arg Leu Arg Asp Gly Gln Leu
       210                 215                 220
   Val Ser Gln Arg Ala Ile Pro Pro Arg Ser Ile Arg Gly Asp Gln Ile
   225                 230                 235                 240
   Ala Trp Val Glu Gly His Glu Pro Gly Cys Arg Ser Ile Gly Ala Leu
                   245                 250                 255
   Met Ala His Val Asp Ala Val Ile Arg His Cys Ala Gly Arg Leu Gly
               260                 265                 270
   Ser Tyr Val Ile Asn Gly Arg Thr Lys Ala Met Val Ala Cys Tyr Pro
           275                 280                 285
   Gly Asn Gly Leu Gly Tyr Val Arg His Val Asp Asn Pro His Gly Asp
       290                 295                 300
   Gly Arg Cys Ile Thr Cys Ile Tyr Tyr Leu Asn Gln Asn Trp Asp Val
   305                 310                 315                 320
   Lys Val His Gly Gly Leu Leu Gln Ile Phe Pro Glu Gly Arg Pro Val
                   325                 330                 335
   Val Ala Asn Ile Glu Pro Leu Phe Asp Arg Leu Leu Ile Phe Trp Ser
               340                 345                 350
   Asp Arg Arg Asn Pro His Glu Val Lys Pro Ala Tyr Ala Thr Arg Tyr
           355                 360                 365
   Ala Ile Thr Val Trp Tyr Phe Asp Ala Lys Glu Arg Ala Ala Ala Lys
   370                 375                 380
   Asp Lys Tyr Gln Leu Ala Ser Gly Gln Lys Gly Val Gln Val Pro Val
   385                 390                 395                 400
   Ser Gln Pro Pro Thr Pro Thr
                   405
<210> 3
   <211> 20
   <212> DNA
   <213> Künstliche Sequenz
<220>
   <223> Beschreibung der künstlichen Sequenz: primer
<400> 3
   ctgtccagga gtgcctagtg 20
<210> 4
   <211> 18
   <212> DNA
   <213> Künstliche Sequenz
<220>
   <223> Beschreibung der künstlichen Sequenz: primer
<400> 4
   gggctggcag tggtagag 18

## Beschreibung

Die vorliegende Erfindung betrifft ein Verfahren zur *In*-*vitro*-Diagnose thromboembolischer Erkrankungen und/oder koronarer Herzerkrankungen, wobei das Nukleotid in Position 470 einer für das menschliche EGLN2-Protein codierenden Nukleinsäure oder die Aminosäure in Position 58 des menschlichen EGLN2-Proteins einer Probe aus einer Person bestimmt wird.

EGLN2, das aufgrund seiner HIF-Prolylhydroxylaseaktivität auch unter dem Namen Prolylhydroxylasedomäne-haltiges Protein 1 (PHD1) bekannt ist, gehört zu einer Gruppe nahe verwandter Proteine der Egl-Nine-Genfamilie, die eine aus fünf codierenden Exons bestehende konservierte genomische Struktur aufweist. Bei HIF (Hypoxie-induzierbarer Faktor) handelt es sich um einen Transkriptionsregulator, dem in vielen Aspekten der Sauerstoffhomöostase eine Schlüsselrolle zukommt, wobei jedoch der Beitrag der EGLN-Isoformen EGLN1 (PHD1), EGLN2 (PHD2) und EGLN3 (PHD3) zur physiologischen Regulation von HIF noch ungewiß ist (Appelhoff, R. J. et al. (2004) J. Biol. Chem., 279, 38458-38465, Nr. 37). Es liegen Berichte vor, nach denen alle EGLN-Isoformen ein unterschiedliches zellspezifisches und induzierbares Verhalten zeigen, was Flexibilität bei der Regulation der HIF-Reaktion auf Hypoxie gestatten sollte. Dies würde bedeuten, daß die spezifische pharmakologische Hemmung eines bestimmten EGLN-Isoenzyms das Potential zur selektiven Modulation der HIF-Reaktion besitzen könnte, die bei therapeutischen Anwendungen von Nutzen wäre (Appelhoff, R. J. et al. (2004), supra). Die Hemmung von EGLN2 sollte beispielsweise unter Ruhebedingungen die HIF-Reaktion breit über eine Reihe von Zellarten aktivieren. Im Gegensatz dazu sollte durch spezifische Hemmung von EGLN3 die Reaktion auf Hypoxie in bestimmten Geweben, die hohe Expressionsniveaus des Enzyms aufweisen, verstärkt werden (Appelhoff, R. J. et al. (2004), supra). Dadurch könnte eine Behandlung ischämischer/hypoxischer Erkrankungen ermöglicht werden. Im Gegensatz zu EGLN2 und EGLN3 ist über die physiologische Rolle von EGLN1 nur wenig bekannt.

Zum besseren Verständnis einer möglichen Beteiligung von EGLN2 an Auftreten und Verlauf koronarer Herzerkrankungen wurden bei einer hinsichtlich einer Variation im EGLN2-Gen in Position 470 der unter der Referenznummer NM_053046.2 gemäß der vorliegenden Erfindung veröffentlichten EGLN2-Referenzsequenz gut charakterisierten Patientengruppe Genotyp/Phänotyp-Assoziationsanalysen durchgeführt. Unterschiedliche genetische Varianten des EGLN2-Gens sind bereits als SNPs (Single Nucleotide Polymorphisms [= Einzelnukleotidpolymorphismen]) bekannt und veröffentlicht unter http://www.ncbi.nlm.nih.gov/SNP/snp ref.cgi?locusId=112398).

Überraschenderweise stellte sich nun heraus, daß eine Variation des Nukleotids in Position 470, insbesondere von Cytidin zu Thymidin, einer für das menschliche EGLN2-Protein codierenden Nukleinsäure oder der Aminosäure in Position 58, insbesondere von Serin zu Leucin, des menschlichen EGLN2-Proteins, mit dem Auftreten thromboembolischer Erkrankungen und/oder koronarer Herzerkrankungen korreliert.

Daher betrifft ein Gegenstand der vorliegenden Erfindung eine *In-vitro-*Diagnose thromboembolischer Erkrankungen und/oder koronarer Herzerkrankungen, wobei das Nukleotid in Position 470 einer für das menschliche EGLN2-Protein codierenden Nukleinsäure oder die Aminosäure in Position 58 des menschlichen EGLN2-Proteins einer Probe aus einer Person oder einem Patienten bestimmt wird.

bei der thromboembolischen Erkrankung handelt es sich um einen Schlaganfall, ein prolongiertes reversibles ischämisches neurologisches Defizit (PRIND) und/oder eine transitorische ischämische Attacke (TIA) und bei der koronaren Herzerkrankung handelt es sich um einen Myokardinfarkt.

Insbesondere besteht ein höheres Risiko eines Schlaganfalls, eines PRIND und/oder einer TIA, wenn das Nukleotid in Position 470 als Thymidin in der chromosomalen DNA oder Uridin in der mRNA oder die Aminosäure in Position 58 als Leucin bestimmt wird. Falls jedoch das Nukleotid in Position 470 als Cytidin oder die Aminosäure in Position 58 als Serin bestimmt wird, besteht ein höheres Risiko eines Myokardinfarkts, insbesondere eines frühzeitigen Myokardinfarkts.

Der Begriff "EGLN2-C470C" bezieht sich im Sinne der vorliegende Erfindung auf diejenige Gruppe von Individuen, die auf beiden Allelen des für EGLN2 codierenden Gens in Position 470 der Referenzsequenz NM_053046.2 Cytidin aufweisen, was zur Aminosäure Serin in Position 58 des entsprechenden Proteins führt. Diese Individuen sind hinsichtlich dieser EGLN2-Variante homozygot. Folglich bezieht sich der Begriff "EGLN2-C470T" auf diejenige Gruppe von Individuen, die auf einem Allel des für EGLN2 codierenden Gens Cytidin aufweisen, was zu Serin in Position 58 des entsprechenden Proteins führt, und auf dem anderen Allel des für EGLN2 codierenden Gens Thymidin aufweisen, was zu Leucin in Position 58 des entsprechenden Proteins führt. Diese Individuen sind hinsichtlich dieser EGLN2-Variante heterozygot.

Die Nukleinsäuresequenz der für das menschliche EGLN2-Protein codierenden Referenzsequenz weist vorzugsweise die Nukleinsäuresequenz der SEQ ID NO: 1 und die Aminosäuresequenz des menschlichen EGLN2-Proteins vorzugsweise die Aminosäuresequenz der SEQ ID NO: 2, auf. Die vorliegende Erfindung umfaßt jedoch auch andere Varianten des menschlichen EGLN2 sowie die nichtmenschlichen Homologen davon, wie beispielsweise EGLN2-Homologe aus anderen Säugem oder die EGLN2-Homologe aus *Caenorhabdidis elegans,* Maus oder Ratte, vorausgesetzt, daß in der Position 470 der Referenzsequenz entprechenden Position ein Nukleotidaustausch von Cytidin zu Thymidin und/oder in der Position 58 der Referenzsequenz entsprechenden Position ein Aminosäureaustausch von Serin zu Leucin vorliegt, und ferner vorausgesetzt, daß das entsprechende Protein eine Prolylhydroxylaseaktivität, insbesondere eine HIF-Prolylhydroxylaseaktivität, aufweist. Die Enzymaktivität läßt sich beispielsweise mittels massenspektrometrischer Analyse, womit die Oxidation von Pro, z.B. Pro⁵⁶⁴ von HIF-1α, nachgewiesen werden kann, oder dem Fachmann bekannter enzymatischer Tests bestimmen.

Im allgemeinen läßt sich das spezifische Nukleotid in Position 470 mit einem Nukleinsäuresequenzierungsverfahren, einer massenspektrometrischen Analyse der Nukleinsäure, einem Hybridisierungsverfahren und/oder einem Amplifikationsverfahren bestimmen. Beispiele für ein Nukleinsäuresequenzierungsverfahren sind Pyrosequenzierung und/oder Sequenzierung mit Hilfe von radioaktiven und/oder fluoreszenzmarkierten Nukleotiden. Beispiele für das Hybridisierungsverfahren sind die Southern-Blot-Analyse, die Northem-Blot-Analyse und/oder ein Hybridisierungsverfahren auf einem DNA-Mikroarray. Beispiele für ein Amplifikationsverfahren sind eine TaqMan-Analyse, eine Differential-RNA-Display-Analyse und/oder eine Repräsentationsdifferenzanalyse (Shi M. M. (2002) Am J Pharmacogenomics., 2(3), 197-205; Kozian & Kirschbaum (1999) Trends Biotechnol., 17(2), 73-8.).

Weiterhin läßt sich die Aminosäuresequenz in Position 58 mit einem Verfahren zur Messung der Menge des spezifischen Proteins bestimmen, wobei das spezifische Protein an Position 58 ein Serin oder Leucin aufweist. Beispiele für ein Verfahren zur Messung der Menge des spezifischen Proteins sind eine Westem-Blot-Analyse und/oder ein ELISA-Test.

Bei einer Probe zum Nachweis der jeweiligen Variante handelt es sich beispielsweise um eine Zelle, ein Gewebe und/oder eine Körperflüssigkeit, insbesondere in zellulären Bestandteilen des Bluts, Endothelzellen oder glatten Muskelzellen. Vorzugsweise wird die Probe zur Isolierung und/oder Aufreinigung der Nukleinsäuren oder von chromosomaler DNA oder der Proteine der Probe für die weitere Analyse mit dem Fachmann bekannten herkömmlichen Verfahren vorbehandelt.

In einem optionalen weiteren Schritt läßt sich die Gefahr für eine Person, an einem Schlaganfall, PRIND, TIA und/oder Myocardinfarkt zu leiden, bestimmen, wie in den Beispielen gezeigt.

In einem weiteren Schritt läßt sich die Dosierung eines Arzneimittels gegen eine thromboembolische Erkrankung und/oder eine koronare Herzerkrankung bestimmen, wobei dieses Aspekt nicht von der Erfindung umfasst wird.
Im allgemeinen läßt sich die gefundene genetische Variation im EGLN2-Gen im Sinne der vorliegenden Erfindung als genetischer Marker zur Beurteilung des Risikos und/oder der vorbeugenden Behandlung einer thromboembolischen Erkrankung und/oder koronarer Herzerkrankung (auch unter dem Namen "Herz-Kreislauf-Erkrankung" bekannt), insbesondere eines frühzeitigen Myokardinfarkts, eines Schlaganfalls, von PRIND, TIA und/oder koronaren Herzerkrankungen, einsetzen.

Weiterhin läßt sich die genetische Variation im Sinne der vorliegenden Erfindung als genetischer Marker zur Anpassung der Dosierung eines therapeutischen Wirkstoffs zur Behandlung einer Person, eines Individuums oder Patienten und/oder zur Identifizierung von an klinischen Versuchsstudien teilnehmenden oder dafür ausgewählten Personen, Individuen oder Patienten mit einem erhöhten Risiko einer thromboembolischen Erkrankung und/oder koronaren Herzerkrankung, insbesondere eines frühzeitigen Myokardinfarkts, eines Schlaganfalls, von PRIND, TIA und/oder koronaren Herzerkrankungen, einsetzen. Ebenso läßt sich die genetische Variation im Sinne der vorliegenden Erfindung zur Bewertung der Toleranz, Sicherheit und Wirksamkeit eines pharmazeutischen Wirkstoffs bei einer bestimmten Person, einem bestimmten Individuum oder Patienten oder bei der Identifizierung der Person, des Individuums oder Patienten, die bzw. der für eine jeweilige Behandlung der genannten Erkrankungen in Frage kommt, einsetzen.

Darüber hinaus läßt sich die genetische Variation im Sinne der vorliegenden Erfindung auch als Teil eines Tests mit hohem Durchsatz zum Nachweis und zur Bewertung pharmazeutisch wirksamer Verbindungen bei der Behandlung der genannten Erkrankungen verwenden.

Die vorliegende Erfindung kann auch jeweils zur Identifizierung von Risikofaktoren für die genannten Erkrankungen bei den zu behandelnden oder beratenden Personen, Individuen oder Patienten verwendet werden.

Ein bevorzugtes Verfahren zur Diagnose einer thromboembolischen Erkrankung und/oder koronaren Herzerkrankung im Sinne der vorliegenden Erfindung beinhaltet die folgenden Schritte:
(a) Isolieren einer Nukleinsäuresonde, insbesondere einer DNA-Sonde, aus einer von einer zu untersuchenden Person oder einem zu untersuchenden Patienten stammender Probe;
(b) Amplifizieren des die Position 470 des EGLN2-Gens umfassenden spezifischen Bereichs mit Hilfe von Primern, insbesondere der Primer, wie sie in den Beispielen angegeben sind;
(c) Sequenzieren des amplifizierten Bereichs;
(d) Analysieren des sequenzierten Bereichs sowie
(e) Beurteilen der Gefahr eines Schlaganfalls, PRIND, TIA und/oder einer Myocardinfarkts, insbesondere eines frühzeitigen Myokardinfarkts, der bei Männern unter 55 Jahren und bei Tranen unter 60 Jahren auftritt,
wobei die Probe ein Zelle, ein Gewebe, eine Körperflüssigkeit, ein zellulärer Bestandteil des Bluts, von Endothelzellen oder glatten Muskelzelleneine sein kann.

Ein alternatives Verfahren zur Diagnose einer thromboembolischen Erkrankung und/oder koronaren Herzerkrankung im Sinne der vorliegenden Erfindung beinhaltet die folgenden Schritte:
(a) Isolieren des EGLN2-Proteins aus einer von einer zu untersuchenden Person oder einem zu untersuchenden Patienter, stammenden Probe;
(b) Bestimmen der Aminosäure in Position 58 des EGLN2-Proteins und
(c) Beurteilen der Gefahr einer thromboembolischen Erkrankung und/oder einer koronaren Herzerkrankung, insbesondere eines frühzeitigen Myokardinfarkts, eines Schlaganfalls, von PRIND, TIA und/oder koronaren Herzerkrankungen, wobei die Probe eine Zelle, ein Gewebe, eine Körperflüssigkeit, ein zellulärer Bestandteil des Bluts, von endothelzellen oder glatten Muskelzellen sein kann.

Weitere bevorzugte Schritte sind einzeln oder gemeinsam in den Beispielen angegeben und hiermit durch Bezugnahme auf jeden Schritt aufgenommen.

Mit den folgenden Figuren, Tabellen, Sequenzen und Beispielen soll die vorliegende Erfindung erläutert werden, ohne daß dadurch der Umfang der Erfindung beschränkt wird.

### BESCHREIBUNG DER FIGUREN

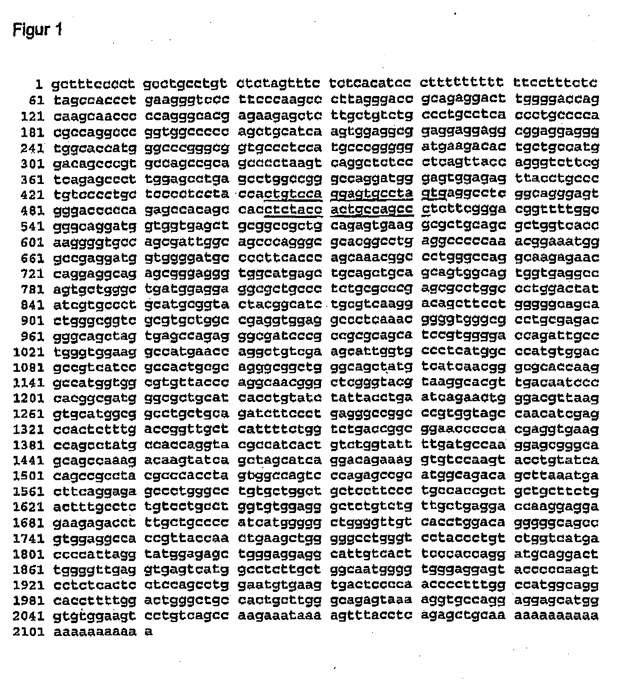

- Figur 1: zeigt die Nukleinsäuresequenz des menschlichen EGLN2-Gens mit der NCBI-Nummer NM_053046. Die zur Amplifikation des Genabschnitts mit der genetischen Variation C→T In Position 470 (in Fettdruck) verwendeten Primer sind unterstrichen.
- Figur 2: zeigt die von der Nukleinsäuresequenz mit der NCBI-Nummer NM_053046 abgeleitete Aminosäuresequenz des menschlichen EGLN2. Die Aminosäureposition 58 im EGLN2-Protein ist in Fettdruck dargestellt.
- Figur 3: zeigt den Einfluß des EGLN2-Genotyps in Position 470 der Referenzsequenz NM_053042.2, der zu Aminosäureaustauschen in Position 58 des EGLN2- Proteins führt, auf das Alter des Auftretens von koronaren Herzerkrankungen in der Patientengruppe. Dabei sind P-Werte unterhalb von 0,05 statistisch relevant.

### BESCHREIBUNG DER SEQUENZEN

- SEQ ID NO: 1: zeigt die Nukleinsäuresequenz des menschlichen EGLN2-Proteins mit der NCBI-Nummer NM_053046.
- SEQ ID NO: 2: zeigt die von der Nukleinsäuresequenz mit der NCBI-Nummer NM_053046 abgeleitete Aminosäuresequenz des menschlichen EGLN2.
- SEQ ID NO 3:: zeigt die erste Primersequenz der Nukleotide 444 - 463 der Referenzsequenz NM_053046.2.
- SEQ ID NO 4:: zeigt die zweite Primersequenz von komplementärer Sequenz der Basen 504 - 521 der Referenzsequenz NM_053046.2.

### BEISPIELE

### SNP-NACHWEIS DURCH SEQUENZIERUNG UND ANALYSE

### Oligonukleotide (Primer) zur Amplifikation:

Zum Nachweis des Nukleotidaustauschs von C zu T in Position 470 in der EGLN2-Sequenz mit der Referenznummer NM_053046.2 wurden die folgenden Primer verwendet:
Primer 1: 5'- CTGTCCAGGAGTGCCTAGTG -3' (Nukleotide 444 - 463 der Referenzsequenz NM_053046.2; SEQ ID NO: 3);
Primer 2: 5'- GGGCTGGCAGTGGTAGAG -3' (komplementäre Sequenz der Basen 504 - 521 der Referenzsequenz NM_053046.2; SEQ ID NO: 4).

### PCR-Protokoll zur Amplifikation:

Die verwendeten Reagentien stammten von Applied Biosystems (Foster City, USA):20 ng genomische DNA; 1 Einheit (Unit) TaqGold DNA-Polymerase; 1x Taq-Polymerase-Puffer; 500µM dNTPs; 2,5mM MgCl₂; je 200nM des Amplifikations-Primerpaars, wie oben gezeigt; H₂O ad 5 µl.

### Amplifikationsprogramm der PCR zur Genotypisierung:

| | |
|---|---|
| 95°C,10 min | x 1 Zyklus |
| | |
| 95°C, 30 s | |
| 70°C, 30 s | x 2 Zyklen; |
| | |
| 95°C, 30 s | |
| 65°C, 30 s | x 2 Zyklen; |
| | |
| 95°C, 30 s | |
| 60°C, 30 s | x 2 Zyklen; |
| | |
| 95°C, 30 s | |
| 56°C, 30 s | |
| 72°C, 30 s | x 40 Zyklen; |
| | |
| 72°C, 10 min | |
| 4°C, 30 s | x 1 Zyklus; |

### Protokoll zur Minisequenzierung und zum Nachweis von SNPs

Die verwendeten Reagentien stammten von Applied Biosystems (Foster City, USA). 2 µl gereinigtes PCR-Produkt, 1,5 µl BigDye-Terminator-Kit, 200nM eines Sequenzierprimers, wie oben gezeigt; H₂O ad 10 µl.

### Amplifikationsprogramm zur Sequenzierung:

| | |
|---|---|
| 96°C, 2 min | x 1 Zyklus; |
| | |
| 96°C, 10 s | |
| 55°C, 10 s | |
| 65°C, 4 min | x 30 Zyklen; |
| | |
| 72°C, 7 min | |
| 4°C, 30 s | x 1 Zyklus; |

### Analyse der Sequenzierunctsprodukte:

Die Sequenzen wurden zunächst zur Gewinnung vorläufiger Daten mit der Sequenz Analyse Software (Applied Biosystems, Foster City, USA) analysiert und dann mit der Software Phred, Phrap, Polyphred und Consed prozessiert. Phred, Phrap, Polyphred und Consed, geschrieben von Phil Green, Washington University (http://www.genome.washington.edu).

### ERGEBNISSE

### Charakteristika der Gruppe von Personen

Tabelle 1 zeigt die Charakteristika der untersuchten Personengruppe.

**Tabelle 1**

| | | n | % |
|---|---|---|---|
| Gesamt | | 2074 | |
| Geschlecht | Weiblich | 603 | 29,07 |
| | Männlich | 1471 | 70,93 |
| Alter | | 61,8 (+/- 10,5) | |
| BMI (Body Mass Index) | | 29,1 (+/- 4,4) | |
| Blutdruck | | 1214 | 58,7 |
| Raucher | | 1372 | 66,41 |
| Typ-II-Diabetes | | 361 | 17,46 |
| Myokardinfarkt | | 830 | 40,59 |
| Schlaganfall | | 145 | 7,01 |

### Häufigkeit und Verteilung der Varianten des EGLN2-Gens

Tabelle 2 zeigt die Häufigkeit und Verteilung der genetischen Varianten des EGLN2-Gens in Position 470 der Referenzsequenz NM_053046.2 in der untersuchten Patientengruppe.

**Tabelle 2**

| | Häufigkeit | Prozentanteil |
|---|---|---|
| EGNL2-C470C (EGNL2 Ser58Ser) | 1253 | 96,31 |
| EGNL2-C470T (EGNL2 Ser58Leu) | 47 | 3,61 |
| EGNL2-T470T (EGNL2 Leu58Leu) | 1 | 0,08 |
| Fehlende Werte | 773 | |

Im folgenden sind nur Individuen mit EGLN2-C470C (EGLN2 Ser58Ser) und EGLN2-C470T (EGLN2 Ser58Leu) berücksichtigt.

### Einfluß der EGLN2-Varianten auf das Auftreten von frühzeitigem Myokardinfarkt

Tabelle 3 zeigt den Einfluß des EGLN2-Genotyps in Position 470 der Referenzsequenz NM_053046.2 auf das Auftreten von frühzeitigem Myokardinfarkt (bei Männern unter 55 Jahren und bei Frauen unter 60 Jahren) und von Schlaganfall/PRIND (prolongiertes reversibles ischämisches neurologisches Defizit)/TIA (transitorische ischämische Attacke) in der untersuchten Patientengruppe. P-Werte unterhalb von 0,05 sind statistisch relevant.

**Tabelle 3**

| | EGLN2 | | p-Wert |
|---|---|---|---|
| | C470C/ / Ser58Ser | C470T / Ser58Leu | |
| Klinischer Parameter | n (%) | n (%) | |
| Patienten mit frühzei-tigem Myokardinfarkt | | | |
| (<55m/60w) | 215 (17,16) | 2(4,26%) | 0,0199 |
| Patienten ohne frühzeitigen Myokardinfarkt | | | |
| (<55m/60w) | 1038(82,84) | 45(95,74) | |
| Patienten mit Schlaganfall/PRIND/TIA | 88 (7,23) | 7(14,89) | 0,0418 |
| Patienten ohne Schlaganfall/PRIND/TIA | 1165 (92,77) | 40 (85,11) | |

### Ergebnisse:

1. Die Patienten mit EGLN2-C470C zeigten im Vergleich zu Patienten mit EGLN2-C470T eine statistisch höhere Häufigkeit von frühzeitigem Myokardinfarkt.
2. Die Patienten mit EGLN2-C470T zeigten im Vergleich zu Patienten mit EGLN2-C470C einen signifikanten Anstieg des Risikos, einen Schlaganfall, PRIND und/oder TIA zu erleiden.

### Einfluß der EGLN2-Varianten auf das Alter der Patienten mit koronaren Herzerkrankungen

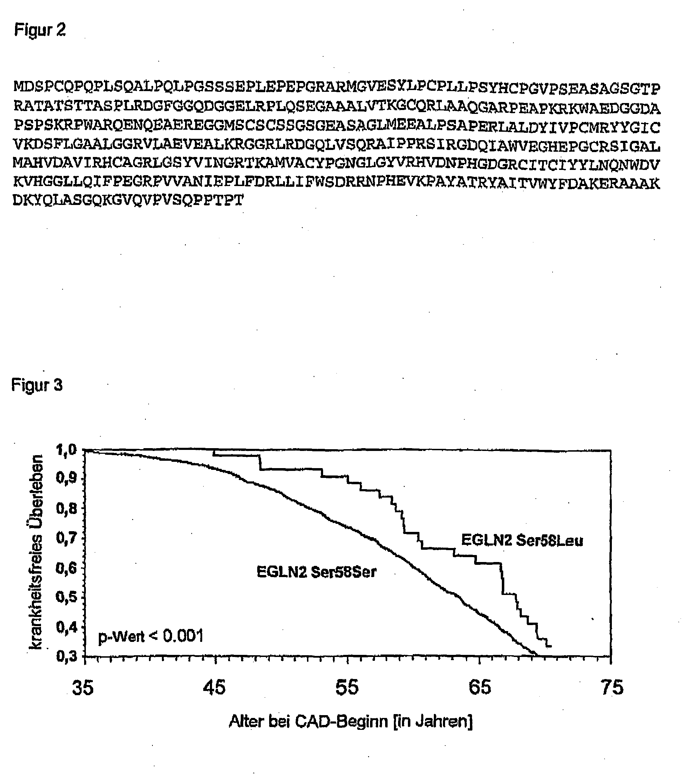

Figur 3 zeigt den Einfluß des EGLN2-Genotyps in Position 470 der Referenzsequenz NM_053042.2 auf das Alter des Auftretens von koronaren Herzerkrankungen in der Patientengruppe.

### Ergebnis:

Es wurde eine signifikante Altersabhängigkeit der Patienten mit EGLN2-C470C (EGLN2 Ser58Ser) für das frühzeitige Auftreten von koronaren Herzerkrankungen im Vergleich zum Alter der Patienten mit EGLN2-C470T (EGLN2 Ser58Leu) festgestellt.

### Schlußfolgerung:

Die oben dargestellten statistisch signifikanten Assoziationen zwischen den genetischen Varianten des für EGLN2 codierenden Gens und/oder des Proteins EGLN2 sind ein deutlicher Hinweis auf die Beteiligung der genetischen Varianten am Auftreten thrombotischer Erkrankungen und/oder koronarer Herzerkrankungen. Folglich stellen die genetischen Varianten biologische Marker für die Prognose thrombotischer Erkrankungen und/oder koronarer Herzerkrankungen, insbesondere für die Prognose von frühzeitigem Myokardinfarkt und/oder Schlaganfall, PRIND und/oder TIA, dar.
SEQ ID NO: 1
SEQ ID NO: 2
SEQ ID NO: 3
   1 CTGTCCAGGA GTGCCTAGTG
SEQ ID NO: 4
   1 GGGCTGGCAG TGGTAGAG

## Patentansprüche

1. Verfahren zur In-vitro-Diagnose thromboembolischer Erkrankungen und/oder koronarer Herzerkrankungen, wobei das Nukleotid in Position 470 einer für das menschliche EGLN2-Protein codierenden Nukleinsäure oder die Aminosäure in Position 58 des menschlichen EGLN2-Proteins einer Probe aus einer Person bestimmt wird, wobei die thromboembolische Erkrankung ausgewählt ist aus Schlaganfall, prolongiertes reversibles ischämisches neurologisches Defizit (PRIND) und/oder transitorische ischämische Attacke (TIA) und wobei es sich bei der koronaren Herzerkrankung um Myokardinfarkt handelt.

2. Verfahren nach Anspruch 1, wobei als Risikofaktor für Schlaganfall, PRIND und/oder TIA das Nukleotid in Position 470 als Thymidin in der chromosomalen DNA oder Uridin in der mRNA oder die Aminosaure in Position 58 als Leucin bestimmt wird.

3. Verfahren nach Anspruch 2, wobei als Risikofaktor für Myokardinfarkt, insbesondere frühzeitigen Myokardinfarkt, das Nukleotid in Position 470 als Cytidin oder die Aminosäure in Position 58 als Serin bestimmt wird, wobei frühzeitiger Myokardinfarkt so definiert ist, dass er bei Männern unter 55 Jahren und bei Frauen unter 60 Jahren auftritt.

4. Verfahren nach einem der Ansprüche 1-3, wobei die für das menschliche EGLN2-Protein codierende Nukleinsäure die Nukleotidsequenz der SEQ ID NO: 1 aufweist.

5. Verfahren nach einem der Ansprüche 1-3, wobei das menschliche EGLN2-Protein die Aminosäuresequenz der SEQ ID NO: 2 aufweist.

6. Verfahren nach einem der Anspruche 1, 2 oder 4, wobei das Nukleotid in Position 470 mit einem Verfahren, ausgewählt aus der Gruppe bestehend aus einem Nukleinsäuresequenzierungsverfahren, einer massenspektrometrischen Analyse der Nukleinsäure, einem Hybridisierungsverfahren und/oder einem Amplifikationsverfahren, bestimmt wird.

7. Verfahren nach Anspruch 6, wobei das Nukleinsäuresequenzierungsverfahren ausgewählt ist aus der Gruppe, ausgewählt aus Pyrosequenzierung und/oder Sequenzierung mit Hilfe von radioaktiven und/oder fluoreszenzmarkierten Nukleotiden.

8. Verfahren nach Anspruch 6, wobei das Hybridisierungsverfahren ausgewählt ist aus der Gruppe bestehend aus Southern-Blot-Analyse, Northern-Blot-Analyse und/oder einem Hybridisierungsverfahren auf einem DNA-Mikroarray.

9. Verfahren nach Anspruch 6, wobei das Amplifikationsverfahren ausgewählt ist aus der Gruppe bestehend aus einer TaqMan-Analyse, einer Differential-RNA-Display-Analyse und/oder einer Repräsentationsdifferenzanalyse.

10. Verfahren nach einem der Ansprüche 1-3 und 5, wobei die Aminosäuresequenz in Position 58 mit einem Verfahren zur Messung der Menge des spezifischen Proteins bestimmt wird, wobei das spezifische Protein an Position 58 ein Serin oder Leucin aufweist.

11. Verfahren nach Anspruch 10, wobei die Menge des spezifischen Proteins mit einem Verfahren, ausgewählt aus der Gruppe bestehend aus einer Western-Blot-Analyse und/oder einem ELISA-Test, gemessen wird.

12. Verfahren nach einem der Ansprüche 1-11, wobei die Probe ausgewählt ist aus der Gruppe bestehend aus einer Zelle, einem Gewebe und/oder einer Körperflüssigkeit.

13. Verfahren nach einem der Ansprüche 1-12, das die folgenden Schritte umfasst:
(a) Isolieren einer Nukleinsäuresonde, insbesondere einer DNA-Sonde, aus einer von einer zu untersuchenden Person stammenden Probe;
(b) Amplifizieren des die Position 470 des EGLN2-Gens umfassenden spezifischen Bereichs mit Hilfe von Primern;
(c) Sequenzieren des amplifizierten Bereichs;
(d) Analysieren des sequenzierten Bereichs sowie
(e) Beurteilen der Gefahr eines Schlaganfalls, eines PRIND, einer TIA und/oder eines Myokardinfarkts,

14. Verfahren nach Anspruch 13, wobei es sich bei dem Myokardinfarkt um einen frühzeitigen Myokardinfarkt handelt, wobei frühzeitiger Myokardinfarkt so definiert ist, dass er bei Männern unter 55 Jahren und bei Frauen unter 60 Jahren auftritt.

15. Verfahren nach Anspruch 13 oder 14, wobei die Primer ausgewählt sind aus SEQ ID NO: 3 und/oder SEQ ID NO: 4.

16. Verfahren nach einem der Ansprüche 1-3, 5 oder 10-12, das die folgenden Schritte umfasst:
(a) Isolieren des EGLN2-Proteins aus einer aus einer zu untersuchenden Person oder einem zu untersuchenden Patienten stammenden Probe, insbesondere einer Zelle, einem Gewebe, einer Körperflüssigkeit, einem zellulären Bestandteil des Bluts, von Endothelzellen oder glatten Muskelzellen;
(b) Bestimmen der Aminosäure in Position 58 des EGLN2-Proteins und
(c) Beurteilen der Gefahr eines Schlaganfalls, eines PRIND, einer TIA und/oder eins Myocardinfarkts, insbesonde eines frühzeitigen Myokardinfarkts, wobei frühzeitiger Myokardinfarkt so definiert ist, dass er bei Männern unter 55 Jahren und bei Frauen unter 60 Jahren auftritt.

17. Verfahren nach Anspruch 16, wobei es sich bei dem Myokardinfarkt um einen frühzeitigen Myokardinfarkt, wie in Anspruch 16 definiert, handelt.

## Claims

1. A method for the in vitro diagnosis of thromboembolic and/or coronary heart diseases, wherein the nucleotide at position 470 of a nucleic acid coding for the human EGLN2 protein or the amino acid at position 58 of the human EGLN2 protein of a sample of a person is determined, wherein the thromboembolic disease is selected from stroke, prolonged reversible ischemic neurological deficit (PRIND) and/or transitoric ischemic attack (TIA) and wherein the coronary heart disease is myocardial infarction.

2. The method of claim 1, wherein the nucleotide at position 470 is determined as thymidine in the chromosomal DNA or uridine in the mRNA or the amino acid at position 58 is determined as leucine for a risk of stroke, PRIND and/or TIA.

3. The method of claim 2, wherein the nucleotide at position 470 is determined as a cytidine or the amino acid at position 58 is determined as serine for a risk of myocardial infarction, in particular early myocardial infarction, wherein early myocardial infarction is defined as occurring in men under 55 years and in women under 60 years.

4. The method according to any of the claims 1-3, wherein the nucleic acid coding for the human EGLN2 protein has the nucleotide sequence of SEQ ID NO: 1.

5. The method according to any of the claims 1-3, wherein the human EGLN2 protein has the amino - acid sequence of SEQ ID NO: 2.

6. The method according to any of the claims 1, 2 or 4, wherein the nucleotide at position 470 is determined by a method selected from the group consisting of a nucleic acid sequencing method, a mass spectrometric analysis of the nucleic acid, a hybridisation method and/or an amplification method.

7. The method of claim 6, wherein the nucleic acid sequencing method is selected from the group selected from pyrosequencing and/or sequencing with the help of radioactive and/or fluorescence labelled nucleotides.

8. The method of claim 6, wherein the hybridisation method is selected from the group consisting of Southern blot analysis, Northern blot analysis and/or a hybridisation method on a DNA-microarray.

9. The method of claim 6, wherein the amplification method is selected from the group consisting of a TaqMan analysis, a differential RNA display analysis and/or a representational difference analysis.

10. The method according to any of the claims 1-3 and 5, wherein the amino acid sequence at position 58 is determined by a method for measuring the amount of the specific protein, wherein the specific protein has a serine or leucine at position 58.

11. The method according to claim 10, wherein the amount of the specific protein is measured by a method selected from the group consisting of a Western blot analysis and/or an ELISA.

12. The method according to any of the claims 1-11, wherein the sample is selected from the group consisting of a cell, a tissue and/or a body fluid.

13. The method according to any of the claims 1-12 comprising the following steps:
(a) isolating a nucleic acid probe, in particular a DNA probe from a sample from a person that should be investigated;
(b) amplifying the specific region encompassing position 470 of the EGLN2 gene with the help of primers;
(c) sequencing the amplified region;
(d) analysing the sequenced region; and
(e) assessing the risk for a stroke, a PRIND, a TIA and/or a myocardial infarction.

14. The method according to claim 13, wherein the myocardial infarction is an early myocardial infarction, wherein early myocardial infarction is defined as occurring in men under 55 years and in women under 60 years.

15. The method according to claim 13 or 14, wherein the primers are selected from SEQ ID NO: 3 and/or SEQ ID NO: 4.

16. The method according to any of the claims 1-3, 5 or 10-12 comprising the following steps:
(a) isolating the EGLN2 protein from a sample, in particular a cell, tissue, body fluid, a cellular component of the blood, endothelial cells or smooth muscle cells, from a person or patient that should be investigated;
(b) determining the amino acid at position 58 of the EGLN2 protein; and
(c) assessing the risk for a stroke, a PRIND, a TIA and/or a myocardial infarction, in particular for an early myocardial infarction, , wherein early myocardial infarction is defined as occurring in men under 55 years and in women under 60 years.

17. The method according to claim 16, wherein the myocardial infarction is an early myocardial infarction as defined in claim 16.

## Revendications

1. Procédé pour le diagnostic in vitro de maladies thromboemboliques et/ou de maladies coronariennes, où on détermine le nucléotide en position 470 d'un acide nucléique codant pour la protéine EGLN2 humaine ou l'acide aminé en position 58 de la protéine EGLN2 humaine dans un échantillon d'une personne, la maladie thromboembolique étant choisie parmi une attaque, un déficit neurologique ischémique réversible prolongé (PRIND) et/ou une attaque ischémique transitoire (TIA) et où il s'agit, pour la maladie coronarienne, d'un infarctus du myocarde.

2. Procédé selon la revendication 1, où on détermine, comme facteur de risque pour l'attaque, le PRIND et/ou la TIA, le nucléotide en position 470 comme étant la thymidine dans l'ADN chromosomique ou l'uridine dans l'ARNm ou l'acide aminé en position 58 comme étant la leucine.

3. Procédé selon la revendication 2, où on détermine, comme facteur de risque pour l'infarctus du myocarde, en particulier un infarctus du myocarde précoce, le nucléotide en position 470 comme étant la cytidine ou l'acide aminé en position 58 comme étant la sérine, l'infarctus du myocarde précoce étant défini comme survenant chez les hommes avant 55 ans et chez les femmes avant 60 ans.

4. Procédé selon l'une quelconque des revendications 1-3, où l'acide nucléique codant pour la protéine EGLN2 humaine présente la séquence de nucléotides de la séquence SEQ ID NO : 1.

5. Procédé selon l'une quelconque des revendications 1-3, où la protéine EGLN2 humaine présente la séquence d'acides aminés de la séquence SEQ ID NO : 2.

6. Procédé selon l'une quelconque des revendications 1, 2 ou 4, où le nucléotide en position 470 est déterminé par un procédé choisi dans le groupe constitué par un procédé de séquençage d'acide nucléique, une analyse spectrométrique de masse de l'acide nucléique, un procédé d'hybridation et/ou un procédé d'amplification.

7. Procédé selon la revendication 6, où le procédé de séquençage d'acide nucléique est choisi dans le groupe choisi parmi le pyroséquençage et/ou le séquençage à l'aide de nucléotides radioactifs et/ou marqués par fluorescence.

8. Procédé selon la revendication 6, où le procédé d'hybridation est choisi dans le groupe constitué par l'analyse Southern-Blot, l'analyse Northern-Blot et/ou un procédé d'hybridation sur une puce à ADN.

9. Procédé selon la revendication 6, où le procédé d'amplification est choisi dans le groupe constitué par une analyse TaqMan, une analyse par affichage différentiel d'ARN et/ou une analyse par différence de représentation.

10. Procédé selon l'une quelconque des revendications 1-3 et 5, où la séquence d'acides aminés en position 58 est déterminée par un procédé pour la mesure de la quantité de protéine spécifique, où la protéine spécifique présente en position 58 une sérine ou une leucine.

11. Procédé selon l'une quelconque des revendications 10, où la quantité de protéine spécifique est mesurée par un procédé choisi dans le groupe constitué par une analyse Western-Blot et/ou un test ELISA.

12. Procédé selon l'une quelconque des revendications 1-11, où l'échantillon est choisi dans le groupe constitué par une cellule, un tissu et/ou un liquide corporel.

13. Procédé selon l'une quelconque des revendications 1 à 12, comprenant les étapes suivantes :
(a) isolement d'une sonde d'acide nucléique, en particulier d'une sonde d'ADN, d'un échantillon provenant d'une personne à examiner ;
(b) amplification de la zone spécifique comprenant la position 470 du gène EGLN2 à l'aide d'amorces ;
(c) séquençage de la zone amplifiée ;
(d) analyse de la zone séquencée ainsi que
(e) évaluation du risque d'une attaque, d'un PRIND, d'une TIA et/ou d'un infarctus du myocarde.

14. Procédé selon la revendication 13, où il s'agit, pour l'infarctus du myocarde, d'un infarctus du myocarde précoce, l'infarctus du myocarde précoce étant défini comme survenant chez les hommes avant 55 ans et chez les femmes avant 60 ans.

15. Procédé selon la revendication 13 ou 14, les amorces étant choisies parmi les séquences SEQ ID NO : 3 et/ou SEQ ID NO : 4.

16. Procédé selon l'une quelconque des revendications 1-3, 5 ou 10-12, comprenant les étapes suivantes :
(a) isolement d'une protéine EGLN2 d'un échantillon provenant d'une personnes à examiner ou d'un patient à examiner, en particulier d'une cellule, d'un tissu, d'un liquide corporel, d'un constituant cellulaire du sang, de cellules endothéliales ou de cellules de muscle lisse ;
(b) détermination de l'acide aminé en position 58 de la protéine EGLN2 et
(c) évaluation du risque d'une attaque, d'un PRIND, d'une TIA et/ou d'un infarctus du myocarde, en particulier d'un infarctus du myocarde précoce, l'infarctus du myocarde précoce étant défini comme survenant chez les hommes avant 55 ans et chez les femmes avant 60 ans.

17. Procédé selon la revendication 16, où il s'agit, pour l'infarctus du myocarde, d'un infarctus du myocarde précoce, tel que défini dans la revendication 16.
